## Europäisches Patentamt
## European Patent Office
## Office européen des brevets

(19)

(11) Veröffentlichungsnummer: **0 042 973**
**A1**

(12) # EUROPÄISCHE PATENTANMELDUNG

(21) Anmeldenummer: **81103990.8**

(22) Anmeldetag: **25.05.81**

(51) Int. Cl.³: **A 61 F 5/44**

(30) Priorität: **28.06.80 DE 3024466**

(43) Veröffentlichungstag der Anmeldung:
**06.01.82 Patentblatt 82/1**

(84) Benannte Vertragsstaaten:
**AT BE CH DE FR GB IT LI NL SE**

(71) Anmelder: **Beiersdorf Aktiengesellschaft**
**Unnastrasse 48**
**D-2000 Hamburg 20(DE)**

(72) Erfinder: **Schulte, Dietrich, Dr. Dipl.-Chem.**
**Reichenberger Strasse 11**
**D-2080 Pinneberg(DE)**

(72) Erfinder: **Schmidt, Erich, Dr.**
**Steinbachtal 39a**
**D-8700 Würzburg(DE)**

(72) Erfinder: **Sinnen, Marike**
**Fröbelstrasse 7**
**D-2080 Pinneberg(DE)**

(72) Erfinder: **Hahn, Siegfried**
**Alsterwiesen 48**
**D-2359 Henstedt-Ulzburg(DE)**

(54) **Colostomiebeutel.**

(57) Colostomiebeutel mit einer Öffnung zur Anlage an das Stoma und einer die Öffnung umgebenden Kleibstoffbeschichtung zur Befestigung auf der das Stoma umgebenden Haut, bei welchem auf der Beutelaußenseite in Gegenüberlage zur Öffnung für das Stoma eine dicht verschließbare Öffnung (4) angebracht ist. Vorzugsweise ist zum Verschließen der Öffnung (4) eine Verschlußlasche (5) aus selbstklebend ausgerüsteter Folie, die mit einer lösbaren Abdeckfolie (7) versehen ist, vorgesehen.

Fig. 2

EP 0 042 973 A1

0042973

Beiersdorf AG
Hamburg

Colostomiebeutel.

Die vorliegende Erfindung betrifft einen Colostomiebeutel,
d.h. einen Auffangbeutel für Ausscheidungen bei Menschen, die
einen künstlichen Darmausgang (Colostomie) gelegt bekommen
haben, und bei denen nach Anlegen des Beutels von außen her
gleichsam durch den Beutel hindurch Handlungen im Darm-
Inneren vorgenommen werden müssen, damit der Ausscheidungsvorgang stattfinden kann. Während dieses Vorgangs soll der
ursprünglich durchgängige Colostomiebeutel aber hermetisch
(flüssigkeits- und geruchsdicht) verschlossen sein.

Das Anlegen eines künstlichen Darmausganges ist heutzutage ein gängiges chirurgisches Verfahren, wenn bei dem
betreffenden Patienten die Entfernung des Dickdarms oder
von Teilen desselben medizinisch angezeigt ist. Dabei wird
das verbleibende Darmende ("Stoma") so an der Bauchdecke
operativ befestigt, daß an dieser Stelle eine Darmentleerung

BAD ORIGINAL

stattfinden kann. Damit von diesem Kunstafter (Anus praeter naturalis) der Darminhalt hygienisch, diskret und auf relativ einfache Weise entfernt werden kann, sind seit längerem Auffangbeutel in Gebrauch, die entweder mittels eines Gürtels, der um den Bauch gelegt wird, zu befestigen sind, oder die direkt in der Umgebung des Anus praeter auf die Bauchdecke geklebt werden. Alles, was an Stuhl, an Darmgasen oder an Absonderungen der Darmschleimhaut ausgeschieden wird, kann bei Vorliegen eines Anus praeter in dem dicht abschließenden und zweckmäßig aus geruchsdichtem Folien-Material hergestellten Beutel aufgefangen werden; nach dem Entleerungsvorgang oder sobald der Beutel angemessen gefüllt ist, wird er vom Körper abgenommen und mitsamt dem Inhalt auf geeignete Weise beseitigt. Nach der Reinigung des Bereiches um den Anus praeter, der auch kurz "Stoma" genannt wird, kann ein neuer Beutel aufgebracht werden, der den Stuhl bei der nächsten Entleerung auffängt usw.

Ein in der beschriebenen Weise aufgebrachter Auffangbeutel gibt dem Stoma-Träger gute Sicherheit, daß eine Darmentleerung jederzeit vonstatten gehen kann ohne Wahrnehmung durch die Umgebung. Bei vielen Betroffenen erfolgt die Entleerung nämlich unkontrolliert und über längere Zeit hinweg, wohingegen andere ihren Darm dahingehend beeinflussen können, daß nur zu ganz bestimmten Zeiten des Tages eine Entleerung stattfindet. Diese Fähigkeit ist außer von einem gewissen Maß an Willen und Übung auch von der Zusammensetzung der Nahrung sowie von der Einhaltung fester Lebensrhythmen abhängig.

Zur Unterstützung der Regelmäßigkeit in der Stuhlentleerung wird besonders in den V.St.A. die sog. Irrigations- oder Spülmethode angewendet, die sich allerdings in Europa bislang noch nicht recht durchsetzen konnte. Dabei wird mittels geeigneter Vorrichtungen eine Spülflüssigkeit durch die Stoma-Öffnung in den Darm eingebracht, die diesen

BAD ORIGINAL

0042973

-3-

durchspült, seinen Inhalt aufrührt und den Darm zur Entleerung anreizt. Vorrichtungen für derartige Darmspülungen sind z.B. beschrieben in den US-PS 2 331 226, 3 055 365 und DE-AS 2 511 815, bei denen jeweils die Zuführung der Irrigatorflüssigkeit durch eine Vorrichtung geschieht, welche mit einem Auffangsystem (wie z.B. Auffangbeutel, Rohrsystem zur Ableitung direkt in die Toilette usw.) zu einer Einheit verbunden ist. Nach Befestigung dieses kombinierten Systems im Bereich der Stoma-Öffnung kann also die Spülflüssigkeit von außen, z.B. aus einem höher gelegenen Gefäß durch Eigendruck in den Darm geführt werden, dieser damit in der beschriebenen Weise gespült und anschließend sein Inhalt in das Auffanggefäß entleert werden. Auch ist es bekannt, zur leichteren Einfüllung der Flüssigkeit im Inneren des Systems einen Katheter vorzusehen, durch den die Spülflüssigkeit in den Darm eingefüllt wird.

Die genannten Irrigatoreinrichtungen sind für den standardmäßig angelegten Anus praeter gut geeignet und werden in verschiedenen Ländern auch in großem Umfang genutzt.

Neuerdings gibt es jedoch Fortschritte in der Operationstechnik zur Anlage eines Kunstafters, die dazu geführt haben, daß ein Stoma-Träger auch nach seiner Operation kontinent ist.

Beim Magnetverschluß gemäß DE-OS 2 363 563 und 2 447 682 wird ein ringförmiger Permanentmagnet in der Nähe des Darmausganges so implantiert, daß er den Darm umfaßt; wird nun ein Stöpsel aus magnetischem Material auf die über dem implantierten Ring liegende Haut gebracht, so wird die Stoma-Öffnung durch Magnetkräfte dicht verschlossen. Diese elegant anmutende Methode hat jedoch Nachteile, nämlich daß der Körper das ihm fremde Metall abzustoßen bestrebt ist und daß durch den beträchtlichen Druck seitens des Magnet-

BAD ORIGINAL

0042973

- 4 -

verschlusses eine sehr unangenehme Beanspruchung der
Haut auftritt, so daß bei weitem nicht alle Patienten
diese Vorrichtung auf die Dauer ertragen können.

Eine weitere, besonders fortschrittliche und zukunftsweisende Operationsmethode ist die Durchführung einer sog.
Sphinkter-Plastik. Hierbei wird dem Patienten bei der
Verlegung des Darmendes ein wenige cm langes Stück des
gesunden Darms entnommen und unter Zugspannung kurz vor
dem neu vorgesehenen Darmende um diesen Darm gelegt.
Beim Zurückfedern übt das Darmgewebe einen Druck auf den
Darm aus und verschließt diesen in der Funktion eines
künstlichen Sphinkters. Das Darmende selbst wird wie
üblich als Anus praeter zur Bauchdecke geführt und
bildet dort das Stoma. Der durch die Plastik angebrachte
künstliche Sphinkter ist also nur wenige cm von der
Stoma-Öffnung entfernt.

BAD ORIGINAL

0042973

-5-

Aufgrund des künstlichen Sphinkters ist der Colostomist voll kontinent, das bedeutet u.a., daß er nicht während des gesamten Tages einen Auffangbehälter tragen muß. Allerdings entleert sich auch der Darm nicht ohne Zutun, im Gegenteil muß dieser Vorgang künstlich eingeleitet werden, beispielsweise mit Hilfe eines Klistiers. Aufgrund der mit dem Klistier eingeleiteten Flüssigkeit steigt der Druck im Darm so weit an, daß er die Schließwirkung des künstlichen Sphinkters überwindet und der Entleerungsvorgang des Darms stattfindet. Wegen der raschen Reaktion des Darms auf den Einlauf ist es notwendig, bereits vorher einen Auffangbeutel um das Stoma aufzubringen, in den hinein entleert werden kann.

Hier nun bestand ein schwieriges Problem, nämlich daß einerseits das Einleiten einer Einlaufflüssigkeit durch ein Klistier die Zugänglichkeit des Stomas und des Darms erfordert, andererseits aber auch die austretende Darmflüssigkeit aufgefangen werden soll, was wiederum der Forderung nach der Zugänglichkeit des Darms widerspricht. Das Auffangen des Darminhaltes soll außerdem auch so durchgeführt werden, daß der Colostomist nicht durch Gerüche und Flüssigkeitsaustritt belästigt wird, desgleichen soll sichergestellt werden, daß der Darminhalt vollständig in dem dafür vorgesehenen Behälter aufgefangen werden kann. Bei dem durch die Erfindung zu lösenden Problem soll es jedoch nicht allein darum gehen, hier einen Kompromiß zu schließen, vielmehr soll auch sichergestellt sein, daß der Colostomist während des Darmentleerungsvorgangs, der bis zu einer Stunde dauern kann, in seiner Handlungsfreiheit nicht beeinträchtigt ist.

BAD ORIGINAL

-6-

Bekannte Vorrichtungen, die allerdings nicht im Zusammenhang mit dem Anlegen einer Sphinkterplastik stehen, weil derartige Operationen erst in jüngster Zeit durchgeführt werden, können hier nicht eingesetzt werden, weil sie höchstens eine der gestellten Forderungen erfüllen können. So ist z.B. das Einführen eines Klistiers in das Stoma bis in den Bereich des künstlichen Sphinkters hinein dann möglich, wenn der Auffangbehälter nach oben geöffnet ist, was jedoch zu Geruchsbelästigungen führen muß (DE-AS 25 11 815).

Bei einer anderen Ausführungsform einer Vorrichtung zum Auffangen der Darmflüssigkeit wäre zwar die Möglichkeit gegeben,mit Hilfe eines Klistiers das Stoma bzw. den Darm zu erreichen; die hierfür vorgesehene Öffnung des Behälters jedoch kann nicht während des Auffangvorganges dicht abgeschlossen werden, so daß die betroffene Person sich nicht frei bewegen kann und unter Geruchsbelästigungen leiden muß (US-PS 2.928.393).

Die oben geschilderten Nachteile bekannter Vorrichtungen werden durch die Vorrichtung gemäß der Erfindung gelöst, welche im wesentlichen darin zu sehen ist, daß in Gegenüberlage zur Öffnung für das Stoma der Auffangbeutel eine dicht verschließbare Öffnung aufweist.

Weitere vorteilhafte Ausführungsformen gemäß der Erfindung gehen aus den Unteransprüchen hervor[x],wobei besonders wesentlich ist, daß mit Hilfe der vorliegenden Erfindung ein Auffangbehälter geschaffen worden ist, der-ursprünglich durchgängig- während des Auffangvorgangs geruchs- und flüssigkeitsdicht verschlossen ist, wobei der dabei verwendete Verschluß nicht auftragend ausgebildet ist.
Auf diese Art und Weise wird gemäß der Erfindung erreicht,

[x], die Teil dieser Beschreibung sein sollen,

BAD ORIGINAL

0042973

-7-

daß                    die zum Einleiten des Darmentleerungs-
                       vorganges notwendigen Vorgänge durchge-
                       führt werden können,

                       der unmittelbar folgende Auffangvorgang
                       so möglich ist, daß keine Geruchsbelästigung eintritt, wobei die Darmflüssigkeit
                       vollständig aufgefangen wird und nicht
                       wieder entweichen kann

und                    schließlich auch sichergestellt ist, daß
                       der angelegte Auffangbehälter eine genügend lange Zeit am Körper getragen werden
                       kann, ohne daß er die betroffene Person
                       in ihrer Bewegungsfreiheit beeinträchtigt.


Die Erfindung wird nachstehend anhand der Zeichnung
beispielsweise erläutert.

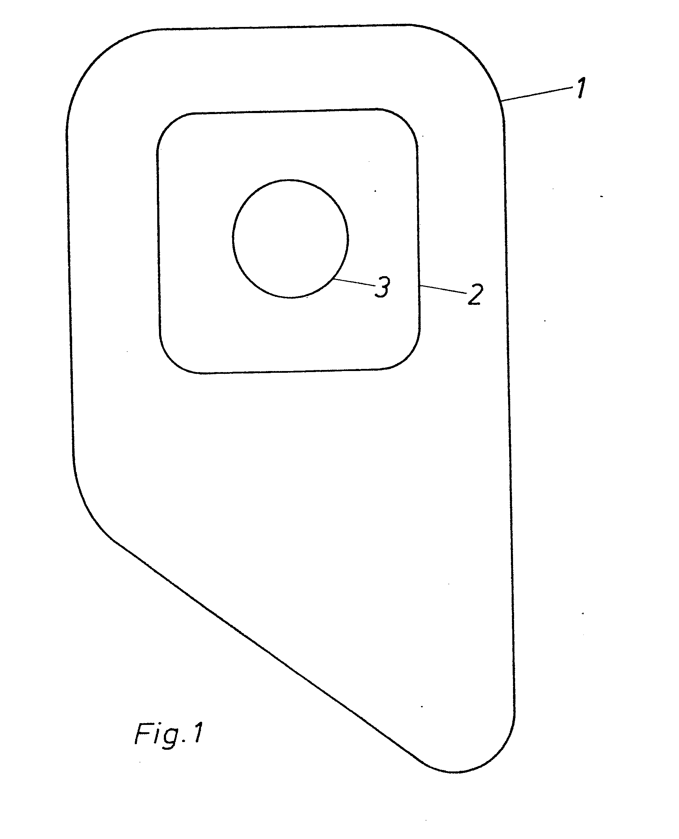

    Fig.1    zeigt einen Colostomiebeutel gemäß der Erfindung,
             gesehen von der dem Körper des Colostomisten
             zugewandten Seite.

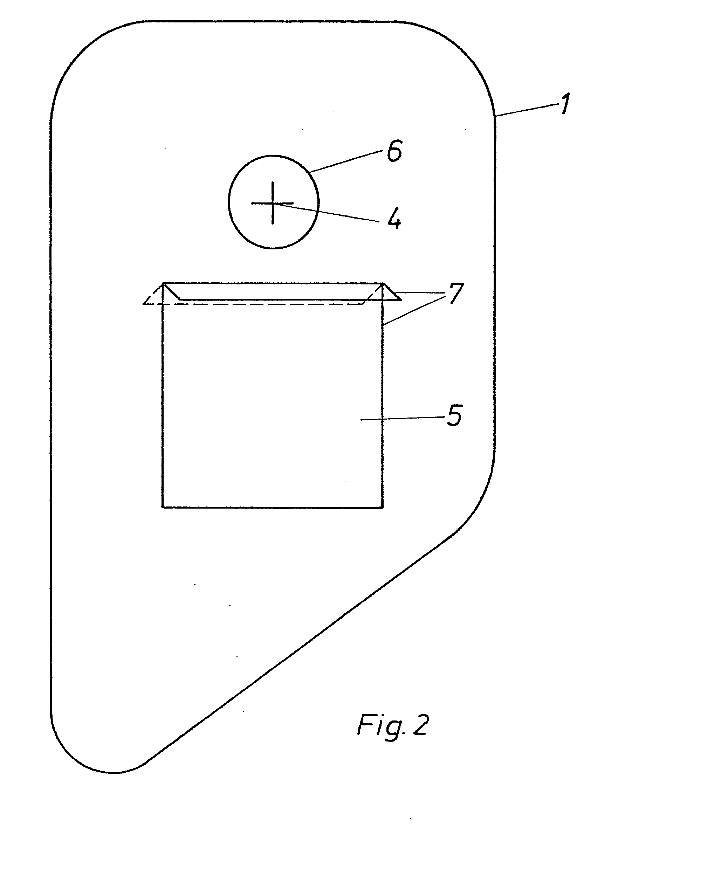

    Fig.2    zeigt einen Colostomiebeutel gemäß der Erfindung,
             gesehen von der dem Körper des Colostomisten
             abgewandten Seite.


Das Auffangsystem besteht im wesentlichen aus einem
Colostomiebeutel 1, welcher mittels eines selbstklebenden
Etiketts 2 auf den Körper des Colostomisten geklebt werden
kann. An der entsprechenden Seite ist der Colostomiebeutel 1
mit einer Öffnung 3 versehen, durch welche der Stuhl aus dem

BAD ORIGINAL

-8-

Darm in den Beutel entleert werden kann (sog.Stomaloch des Beutels). Auf der gegenüberliegenden Beutelseite, d.h. auf der dem Stoma nach dem Anlegen des Beutels abgewandten Seite, ist gemäß der Erfindung eine zweite Öffnung 4 angebracht und mit einem Verschluß 5 versehen, der ein rasches und sicheres Verschließen dieser Öffnung ermöglicht. Die zweite Öffnung 4 muß so gelegen sein, daß sie nach Anlegen des Colostomiebeutels 1 am Körper mit der Öffnung 3 etwa zur Deckung gebracht werden kann,so daß der Colostomist bei angelegtem Beutel von außen her durch die Öffnung 3 und die Öffnung 4 hindurch mit einem Klistier in das Darmende hineingelangen kann. Danach kann die Öffnung 4 mit dem Verschluß rasch und sicher verschlossen werden, so daß nunmehr die Stuhlentleerung beginnen kann.

Der Colostomiebeutel 1 besteht aus zwei geruchsdicht miteinander verschweißten Folien und hat eine Länge von ca. 35 cm und eine Breite von ca. 16 cm mit abgerundeten Ecken. Auf der einen Folienbahn war vor dem Verschweißen paßgenau das mit einem Silikonpapier abgedeckte selbstklebende Etikett 2 aus derselben Folie mit seiner freien Seite aufgeschweißt worden und zentral zu diesem ist durch das Etikett 2 und die Beutelfolie hindurch die runde Öffnung 3 von ca. 40 mm Durchmesser gestanzt worden. Das in der genannten Weise aufgebrachte Etikett 2 ermöglicht später das Aufkleben des Colostomiebeutels 1 auf die Haut. Insofern stellt der Colostomiebeutel 1 eine heute übliche Ausführungsform eines Auffangbehälters dar, wobei die Öffnung für das Stoma mit ihrem Zentrum etwa 8 bis 9 cm von der dem Etikett 2 benachbarten Schmalseite des Colostomiebeutels 1 entfernt auf dessen Längs-Mittellinie liegt.

Auf der zweiten Folienbahn des Colostomiebeutels 1 wird auf deren Außenseite ein rundes Etikett 6 aus anders-

BAD. ORIGINAL

-9-

farbiger Folie aufgeklebt (Durchmesser von etwa 25 mm).
Das Etikett 6 sitzt -bei flach liegendem Beutel- etwa
konzentrisch zur gegenüberliegenden Öffnung 3. Das Etikett
6 und die darunter liegende Beutelfolie werden gemeinsam
sodann mit einem messerartigen Werkzeug (Stanze) so eingeschnitten, daß ein Kreuzschlitz 4 in diesem Folienverbund
entsteht. Durch den Kreuzschlitz 4 und die gegenüberliegende
Öffnung 3 ist der Beutel also "durchgängig", so daß bei
aufgeklebtem Colostomiebeutel 1 der Darm des Colostomisten
zugänglich ist. Etwa 15 mm unterhalb des Etiketts 6 ist
als Verschlußlasche ein ca. 5 cm breiter und 5 cm langer
Streifen 5 angebracht, der aus einer selbstklebenden Folie
derselben Art wie der Beutel besteht.Unterhalb der Öffnung
4 ist der Streifen 5 in einem schmalen Randbereich mit der
Folie des Colostomiebeutels 1 fest verbunden. Es handelt
sich hierbei um einen relativ kleinen Teil des gesamten
Streifens, so daß dieser von dieser Befestigungsstelle frei
nach unten herabhängt. Der auf seiner gesamten später dem
Beutel zugewandten Fläche mit Selbstklebemasse beschichtete
Streifen 5 ist mit einer Abdeckfolie 7 versehen, wobei die
Abdeckfolie etwas länger als der Streifen 5 ausgebildet
ist und am oberen Teil einen vorstehenden Falzrand aufweist.
Mit Hilfe dieses Falzrandes kann die Abdeckfolie 7 vom
Streifen 5 abgezogen werden, und gleichzeitig kann der
Streifen 5 nach oben umgelegt und mit einer einfachen
Handbewegung angedrückt werden, so daß die Öffnung 4
flüssigkeits- und geruchsdicht verschlossen wird.

Im vorangehenden Text ist von Gegenüberlage der Öffnung
für das Stoma und der verschließbaren Öffnung gesprochen
worden, obgleich eine derartige exakte Gegenüberlage zur
Lösung des der Erfindung zugrundeliegenden Problems nicht
zwangsläufig notwendig ist. Wesentlich ist, daß das Problem

BAD ORIGINAL

-10-

auch gelöst werden kann, wenn im Auffangbehälter eine Öffnung für ein Klistier vorgesehen ist, welche von der Bedienungsperson aufgrund der Beweglichkeit des Beutels leicht in Gegenüberlage zum Stoma gebracht werden kann. Beispielsweise könnte die Schweißnaht eines aus zwei Folien hergestellten Auffangbehälters im oberen Bereich, vorzugsweise an einer Ecke, unterbrochen sein, so daß die betroffene Person das Klistier einführen kann und den Auffangbehälter in seiner Form so verändert, daß das Stoma bzw. der Darm zugänglich ist. Nachdem das Klistier entfernt worden ist, kann beispielsweise die Öffnung dadurch verschlossen werden, daß die Ecke, an welcher sich die Öffnung befindet, umgelegt und mit Hilfe eines Klebestreifens abgedichtet wird. Auch wäre es denkbar, daß im Inneren des Behälters vorgesehene Klebeteile nach Herausnahme des Klistiers aufeinandergebracht werden, so daß der Behälter dadurch dicht verschlossen werden kann.

Im Prinzip wird das der Erfindung zugrundeliegende Problem durch eine schnell, sicher und leicht verschließbare Öffnung am Colostomiebeutel gelöst, die sich in Gegenüberlage zum Stomaloch des Beutels befindet. Grundsätzlich können für die Ausbildung der Öffnung auch andere Ausführungsformen als die vorangehend beschriebenen in Frage kommen. Z.B. können Verschlüsse von Kunststoffbeuteln eingesetzt werden, bei denen die Öffnung des Beutels einen verstärkten Rand aufweist und ein flacher kappenförmiger Teil über den verstärkten Rand gedrückt werden kann, so daß durch Zusammenwirken des verstärkten Randes und der Kappe ein einwandfreier Abschluß des Beutels erreicht wird. Dabei kann die Kappe selbst am Kunststoffbeutel durch einen beweglichen Teil befestigt sein, so daß sie vor dem Verschließen nicht verlorengehen kann.

BAD ORIGINAL

- 1-

## Patentansprüche

1. Colostomiebeutel mit einer Öffnung zur Anlage an das Stoma eines Colostomisten und mit einer die Öffnung umgebenden Klebstoffbeschichtung zur Befestigung auf der das Stoma umgebenden Haut des Colostomisten, dadurch gekennzeichnet, daß vorzugsweise in Gegenüberlage zur Öffnung (3) für das Stoma eine dicht verschließbare Öffnung (4) vorgesehen ist.

2. Colostomiebeutel nach Anspruch 1, dadurch gekennzeichnet, daß an der Öffnung (4) eine Verschlußlasche (5) aus selbstklebend ausgerüsteter Folie vorgesehen ist, die ihrerseits mit einer lösbaren Abdeckfolie versehen ist.

3. Colostomiebeutel nach Anspruch 2, dadurch gekennzeichnet, daß ein Teil der Verschlußlasche (5) an dem Colostomiebeutel angeklebt oder mit diesem verschweißt ist.

4. Colostomiebeutel nach einem der Ansprüche 2 oder 3, dadurch gekennzeichnet, daß die Verschlußlasche (5) einseitig vollflächig selbstklebend beschichtet ist.

5. Colostomiebeutel nach einem der Ansprüche 1 bis 4, dadurch gekennzeichnet, daß die dicht vers_chließbare Öffnung (4) rund, quadratisch, drei- oder mehreckig oder als Kreuzschlitz ausgebildet ist.

6. Colostomiebeutel nach einem der Ansprüche 1 bis 5, dadurch gekennzeichnet, daß die Umgebung der verschließbaren Öffnung (4) mittels einer aufgesetzten, aufgeklebten oder aufgeschweißten Folie (6) verstärkt ist und die Verstärkungsfolie (6) deutlich größer als die Öffnung (4) ausgebildet ist.

BAD ORIGINAL

0042973

- 2-

7. Colostomiebeutel nach Anspruch 6, dadurch gekennzeichnet, daß die Verstärkungsfolie (6) eine andere Farbe als die Folie des Colostomiebeutels hat.

- 2-

1/2

Fig.1

Fig. 2

**0042973**

Nummer der Anmeldung

**Europäisches Patentamt**

**EUROPÄISCHER RECHERCHENBERICHT**

**EP 81 10 3990**

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | A 61 F 5/44 |
| X | US - A - 3 618 606 (BROWN) <br> * Zusammenfassung; Abbildungen * <br> -- | 1,3,5 | |
| X | US - A - 3 283 757 (NELSON) <br> * Figuren; Spalte 2, Zeilen 35-42 * <br> -- | 1,5 | |
| D | DE - A - 2 511 815 (HOLLISTER) <br> * Anspruch 1; Figuren * <br> -- | 1,5 | RECHERCHIERTE SACHGEBIETE (Int. Cl.³) <br><br> A 61 F <br> A 61 M |
| A | US - A - 3 570 490 (BERGER) <br> * Spalte 4, Zeilen 7-12; Figuren * <br> ---- | 2-4 | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 02-10-1981 | STEENBAKKER |

EPA form 1503.1  06.78